# EUROPEAN PATENT APPLICATION

(11) **EP 4 129 233 A1**
(43) Date of publication of application: **08.02.2023**
(21) Application number: 21803461.9
(22) Date of filing: 04.03.2021
(51) Int. Cl.: A61F 2/07, A61F 2/856

(54) **COVERED STENT**

(30) Priority: 12.05.2020 CN 202010399195
(71) Applicant: Shanghai MicroPort Endovascular MedTech (Group) Co., Ltd., Shanghai 201318 (CN)
(72) Inventor: ZHU, Qing, Shanghai 201318 (CN); ZHU, Yongfeng, Shanghai 201318 (CN); YUAN, Zhenyu, Shanghai 201318 (CN); ZHAO, Mingjie, Shanghai 201318 (CN); ZHANG, Junli, Shanghai 201318 (CN)
(74) Representative: Marks & Clerk LLP
(86) International application number: PCT/CN2021/078991
(87) International publication number: WO 2021/227612

(57) **Abstract**

The present invention relates to a stent graft, which is used for aortic vascular disease involving the aortic arch (11). The stent graft comprises a main stent (100), a first interface, a second interface, and a first branch stent (310); the main stent (100) comprises a first stent body and a first membrane (120) covering the first stent body; the first branch stent (310) comprises a second stent body and a second membrane (312) covering the second stent body; the first interface is arranged on a side of the main stent (100) and in communication with an inner cavity of the main stent (100); the first branch stent (310) is arranged on the first interface and extends outward, and an end of the second stent body close to the main stent (100) is at least partially connected to the second membrane (312) in a circumferential direction; a recessed area (101) is formed on a side of the main stent (100); the second interface is arranged on the recessed area (101) and in communication with the inner cavity of the main stent (100). The stent graft can be widely used in various groups of people, does not need to be customized for individuals, and has a good therapeutic effect.

## Description

### TECHNICAL FIELD

The present invention relates to the technical field of medical instruments and, more specifically, to a stent graft.

### BACKGROUND

Cardiovascular diseases pose a great threat to human health. In particular, aortic diseases such as aortic aneurysms are one of the most dangerous acute and severe cardiovascular diseases. At present, the treatment methods of aortic disease include drug therapy, surgical treatment, interventional therapy, etc. The principle of interventional therapy is that: the compressible stent is delivered to the diseased part in the blood vessel and then released; after the stent expands in the blood vessel, the diseased part is isolated from the vascular cavity, allowing blood to pass through the stent, thereby protecting the diseased blood vessel, and achieving the purpose of repairing the diseased blood vessel. Interventional therapy is widely used because of its advantages such as small trauma, safety, and effectiveness.

For aortic aneurysms or aortic dissections that do not involve the aortic arch, a straight-tube stent can be used for interventional treatment. For the disease that involves the aortic arch, the use of a straight-tube stent in isolate the lesion will cause a problem that the branch vessels of the aortic arch are covered. Therefore, it is an urgent problem to develop a stent that conforms to the anatomical structure of the aortic arch and avoids covering the branch vessels of the aortic arch.

### SUMMARY OF THE INVENTION

The present invention aims to provide a stent graft, which is adapted to the anatomical structure of the aortic arch, and is configured to be implanted into the aortic arch to isolate the diseased part while avoiding covering the branch blood vessels.

To achieve the above aim, the present invention provides a stent graft, comprising a main stent, a first interface, a second interface, and a first branch stent; the main stent comprises a first stent body and a first membrane covering the first stent body; the first branch stent comprises a second stent body and a second membrane covering the second stent body; the first interface is arranged on a side of the main stent and in communication with an inner cavity of the main stent; the first branch stent is arranged on the first interface and extends outward, and an end of the second stent body close to the main stent is at least partially connected to the second membrane in a circumferential direction; a recessed area is formed on a side of the main stent; the second interface is arranged on the recessed area and in communication with the inner cavity of the main stent.

Optionally, the first interface is disposed outside the recessed area.

Optionally, the recessed area has a depth greater than or equal to 5 mm along a radial direction of the main stent.

Optionally, the first interface comprises a first window and a first positioning ring; the first window is provided on the first membrane of the main stent; the first positioning ring is arranged at the first window and configured to support the first window.

Optionally, the stent graft further comprises a third interface that is arranged in the recessed area of the main stent and in communication with the inner cavity of the main stent; the first interface, the second interface and the third interface are arranged at intervals along an axial direction of the main stent.

Optionally, the second interface comprises a second window and a second positioning ring; the second window is provided on the first membrane of the main stent; the second positioning ring is arranged at the second window and configured to support the second window.

Optionally, the first stent body comprises one first sub-stent body and at least two second sub-stent bodies; the at least two second sub-stent bodies are arranged on two sides of the first sub-stent body along an axial direction thereof; the first sub-stent body and the second sub-stent bodies on the both sides enclose the recessed area; or,
the first stent body includes at least two first sub-stent bodies adjacently arranged and at least two second sub-stent bodies, and the at least two second sub-stent bodies are arranged on two sides of the at least two first sub-stent bodies along an axial direction thereof; the first sub-stent bodies and the second sub-stent bodies on the both sides enclose the recessed area.

Optionally, a gap is formed between the second positioning ring and any one of the first sub-stent bodies; a minimum distance from any point on the second positioning ring to each of the first sub-stent bodies is greater than or equal to 2 mm.

Optionally, the second interface further comprises a connector stent; the connector stent is arranged at the second window and connected with the second positioning ring; an axis of the connector stent is perpendicular to an axis of the main stent.

Optionally, the connector stent has an end connected to the second positioning ring, and a further end extending into the recessed area or the inner cavity of the main stent.

Optionally, the connector stent is extended to a length ranging from 3 mm to 10 mm along an axial direction thereof.

Optionally, the third interface comprises a third window and an embedded stent; the third window is provided on the first membrane of the main stent; the embedded stent is arranged in the inner cavity of the main stent and extends along the axial direction of the main stent; the embedded stent has an end connected to the third window, and a further end connected to the inner cavity of the main stent.

Optionally, the embedded stent comprises a distal end and a proximal end; the distal end of the embedded stent is connected to the third window; an inner diameter of the embedded stent gradually decreases from the distal end to the proximal end thereof.

Optionally, in a cross section of the main stent, an angle between a symmetry axis of the first sub-stent body and a line connecting a center of the main stent with a center of the third interface ranges from 0° to 45°.

Optionally, the main stent has a first side and a second side that are diametrically opposite to each other; the recessed area is provided on the first side;
each of the first sub-stent bodies includes a plurality of V-shaped rods connected end to end in sequence; in the axial direction of the main stent, lengths of the plurality of V-shaped rods of one of the first sub-stent bodies gradually increase from the second side to the first side; and/or
each of the second sub-stent bodies includes a plurality of V-shaped rods connected end to end in sequence; in the axial direction of the main stent, lengths of the plurality of V-shaped rods of one of the second sub-stent bodies gradually increase from the second side to the first side.

Optionally, the main stent has a first side and a second side that are diametrically opposite to each other; the recessed area is provided on the first side; the main stent further comprises a back support extending along the axial direction of the main stent; the back support is arranged on both sides of the recessed area along an axial direction thereof and connected to the second sub-stent body and/or the first membrane.

Optionally, the main stent has a proximal end and a distal end that are opposite to each other; at least a part of the second sub-stent body located at the proximal end and/or the distal end of the main stent is not covered by the first membrane.

Optionally, the main stent has a proximal end and a distal end that are opposite to each other; at least a part of the second sub-stent body located at the proximal end and/or the distal end of the main stent is non-fixedly connected to the first membrane.

Optionally, the stent graft further comprises a second branch stent and/or a third branch stent; the second branch stent is configured to be arranged on the second interface and is detachably connected to the second interface; the third branch stent is configured to be arranged at the third interface and detachably connected to the third interface.

Optionally, the main stent has a proximal end and a distal end that are opposite to each other; the main stent comprises an anterior segment, a middle segment and a posterior segment along a direction from the distal end to the proximal end of the main stent; the first interface and the recessed area are provided on the middle segment, wherein the axial length of the anterior segment has an axial length ranging from 2 cm to 10 cm, and/or the posterior segment has an axial length ranging from 1cm to 20 cm.

Optionally, a diameter of the posterior segment gradually decreases from the distal end to the proximal end of the main stent.

Optionally, a visualization element is provided at each of the first interface, the second interface and the third interface.

Optionally, the second stent body includes a plurality of third sub-stent bodies sequentially arranged along an axial direction of the second stent body;
at least a part of one of the third sub-stent bodies located at a distal end of the second stent body is not covered by the second membrane; or
at least a part of one of the third sub-stent body located at a distal end of the second stent body that is not covered by the second membrane is non-fixedly connected.

Compared with the prior art, the present invention has the following advantages:
When the stent graft is implanted into the patient's aortic arch, the first branch stent provided on the first interface is implanted into a branch blood vessel, so that blood flow can enter from the inner cavity of the first branch stent into the branch blood vessel that conforms to the first branch stent. The recessed area covers the other two branch blood vessels, and the second interface and the third interface are arranged in the recessed area. The connector stent is provided at the second interface, and the second branch stent is inserted into the connector stent to communicate with the inner cavity of the main stent. The second branch stent is implanted into the second branch blood vessel so that blood flow can enter the second branch blood vessel from the inner cavity of the second branch stent. The embedded stent is provided at the third interface, and the separate third branch stent is in communication with the inner cavity of the main stent by being inserted into the embedded stent. The third branch stent is implanted in the third branch blood vessel so that blood flow can enter the third branch blood vessel from the inner cavity of the third branch stent. The stent graft according to the present invention can ensure the smooth blood flow in the branch blood vessels, and avoid the endoleak at the joint between the main stent and the branch stent. In addition, it is simple to position the stent, and the opening of the branch stent can be easily matched with the opening of the branch blood vessel.

### BRIEF DESCRIPTION OF THE DRAWINGS

FIG. 1 is a schematic diagram of a partial structure of a human aorta;
FIG. 2 is a schematic diagram showing a structure of a stent graft according to an embodiment of the present invention, wherein neither the second interface nor the third interface is provided with a branch stent;
FIG. 3 is a schematic diagram showing a partial structure of the stent graft of FIG. 2;
FIG. 4 is a schematic diagram showing the stent graft of FIG. 2 that is implanted into the aorta arch;
FIG. 5 is a schematic diagram showing a structure of a second sub-sent body of the stent graft according to an embodiment of the present invention;
FIG. 6a is a schematic diagram showing a structure of a first sub-sent body of the stent graft according to an embodiment of the present invention;
FIG. 6b is a schematic diagram showing a connection between two first sub-sent bodies of the stent graft according to an embodiment of the present invention;
FIG. 7 is a schematic diagram showing a structure of a first positioning ring of the stent graft according to an embodiment of the present invention;
FIG. 8 is a schematic diagram showing a distal end of the first branch stent of the stent graft according to an embodiment of the present invention;
FIG. 9 is a schematic diagram showing a stent graft that is implanted into the aorta arch according to an embodiment of the present invention, wherein the second interface is provided with a second branch stent, and the third interface is provided with a third branch stent;
FIG. 10a is a schematic diagram showing a partial structure of the main stent of the stent graft according to an embodiment of the present invention, wherein the connector stent is arranged in the inner cavity of the main stent;
Fig. 10b is a schematic diagram showing a partial structure of the main stent of the stent graft according to an embodiment of the present invention, wherein the connector stent is arranged in the recessed area;
Fig. 11 is a schematic diagram showing a radial section of the main stent of the stent graft according to an embodiment of the present invention;
Fig. 12 is a schematic diagram showing a distal end of the main stent of the stent graft according to an embodiment of the present invention;
Fig. 13 is a schematic diagram showing a proximal end of the main stent of the stent graft according to an embodiment of the present invention.

List of reference numerals:
100-main stent
101-recessed area;
111-first sub-stent body, 112-second sub-stent body;
120-first membrane;
130-back support;
211-first positioning ring, 212-visualization element;
221-second positioning ring, 222-connector stent;
231-third window, 232-embedded stent;
310-first branch stent, 320-second branch stent, 330-third branch stent;
311-third sub-stent body, 312-second membrane;
400-restraint wire;
11-aortic arch, 12-ascending aorta, 13-descending aorta, 14-innominate artery, 15- left common carotid artery, 16-left subclavian artery;
S1-anterior segment of the arch, S2-middle segment of the arch, S3-posterior segment of the arch.

### DETAILED DESCRIPTION

To make the objectives, advantages and features of the present invention clearer, the present invention will be further described in detail below with reference to the figures. It is noted that the figures are provided in a very simplified form not necessarily presented to scale, with their only intention to facilitate convenience and clarity in explaining the disclosed embodiments.

As used herein and in the appended claims, the singular forms of "a", "an", and "the" include plural objects, and the plural forms of "plurality" include at least two objects, unless the context clearly dictates otherwise. As used herein and in the appended claims, the term "or" refers to "and/or" unless the context clearly dictates otherwise. In addition, the terms "installed", "connected", "coupled", and "fixed" shall be interpreted broadly, for example, it may be a fixed connection or a detachable connection, or an integrated structure; it may be a mechanical connection or an electrical connection; it may be directly connected or indirectly connected through an intermediate medium, and it may be the internal communication of two components or the interaction relationship between two components. For those of ordinary skill in the art, the specific meanings of the above-mentioned terms in the present disclosure can be understood in specific situations. Throughout the figures, same or similar reference numerals indicate the same or similar elements.

As used herein, a "proximal end" and a "distal end" are relative orientations and relative positions of elements relative to each other from the perspective of a surgeon using the medical instruments. Although the "proximal end" and the "distal end" are not restrictive, the "proximal end" generally refers to the end of the medical instrument that is close to the surgeon during normal operation, while the "distal end" generally refers to the end that first enters the patient.

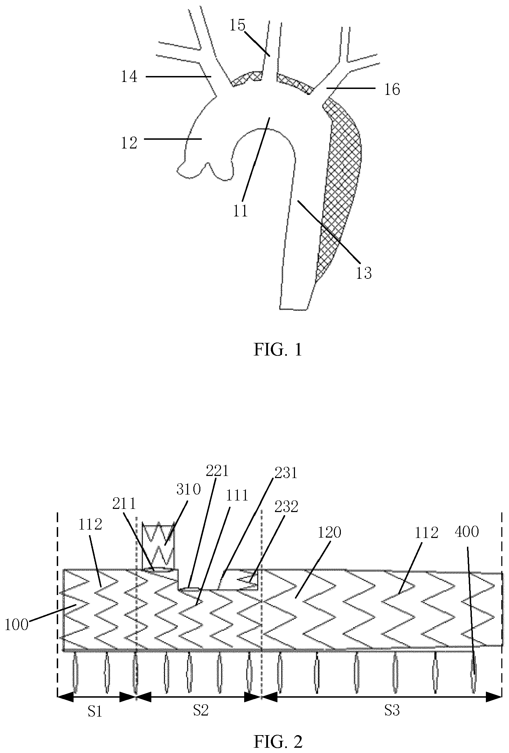

FIG. 1 is a schematic diagram of a partial structure of the human aorta, and mainly shows the aortic arch 11 which is an upper part of the aorta that is curved in an arcuate shape. Taking the orientation shown in FIG. 1 as an example, the part of the aorta adjacent to the left side of the aortic arch 11 is the ascending aorta 12, and the part of the aorta adjacent to the right side of the aortic arch 11 is the descending aorta 13. Three branch blood vessels are on the convex side of the aortic arch 11, which are, from left to right, the innominate artery 14 (that is, the brachiocephalic artery), the left common carotid artery 15 and the left subclavian artery 16. When a disease occurs in the aortic arch 11, a medical stent needs to be implanted in the aortic arch 11 for treatment, and the medical stent should not block the three branch blood vessels.

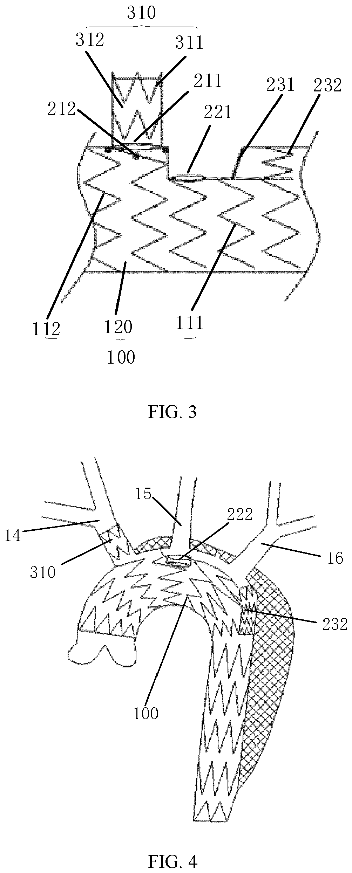

As shown in FIGs. 2 and 3, an embodiment of the present invention provides a stent graft. The stent graft includes a main stent 100, a first interface, a second interface, and a first branch stent 310. The main stent 100 includes a first stent body and a first membrane 120 covering the first stent body. The first branch stent 310 includes a second stent body and a second membrane 312 covering the second stent body. The first interface is arranged on a side of the main stent 100 and in communication with the inner cavity of the main stent 100. The first branch stent 310 is arranged on the first interface and extends outward, and an end of the second stent body close to the main stent 100 is at least partially connected to the second membrane 312 in the circumferential direction. A recessed area 101 is formed on a side of the main stent 100. The second interface is disposed on the recessed area 101 of the main stent 100 and in communication with the inner cavity of the main stent 100.

Referring to FIG. 4, in the embodiment of the present invention, the side of the main stent 100 on which the recessed area 101 is formed is called the first side, and the other side diametrically opposite to the first side is called the second side. When the stent graft is implanted in the aorta, the first side is arranged to conform to the greater curvature side of the aortic arch 11 to provide support to the vessel wall on the greater curvature side of the aortic arch 11, and the second side is arranged to conform to the minor curvature side of the aortic arch 11 to provide support to the vessel wall on the minor curvature side of the aortic arch 11. When the stent graft is implanted in the aortic arch 11, the first branch stent 310 is implanted in a branch blood vessel such as the innominate artery 14 to improve the stability for anchoring of the stent graft in the aorta and avoid displacement.

In the embodiment of the present invention, the main stent 100 and the first branch stent 310 are integrally connected. When the stent graft is implanted in the aortic arch 11, blood flow enters from the inner cavity of the first branch stent 310 into the matching innominate artery 14 which conforms to the first branch stent 310, and endoleaks are avoided while ensuring the patency of the branch vessels. The main stent 100 includes a first stent body and a first membrane 120 covering the first stent body. The first branch stent 310 includes a second stent body and a second membrane 312 covering the second stent body. In an optional implementation, one end of the second stent body close to the main stent 100 is completely connected to the second membrane 312 in the circumferential direction, so that the first branch stent 310 is fixedly arranged at the first interface. In another optional implementation, one end of the second stent body close to the main stent 100 is partially connected to the second membrane 312 in the circumferential direction, so that the first branch stent 310 is movably arranged at the first interface. In addition, it should be understood that the first membrane 120 and the second membrane 312 may be integrally formed, or the first membrane 120 and the second membrane 312 may be separately formed, and then connected to each other by stitching, hot pressing or in other suitable ways.

A recessed area 101 is formed on a side of the main stent 100, and the second interface is disposed on the recessed area 101 of the main stent 100 and in communication with the inner cavity of the main stent 100. When the stent graft is implanted in the aortic arch 11, the recessed area 101 may cover one or two branch blood vessels. The second interface is used to be inserted with a separate second branch stent 320, and the second branch stent 320 is in communication with the inner cavity of the main stent 100. When the stent graft is implanted in the aortic arch 11, the second branch stent 320 is implanted in a branch blood vessel, for example, in the left common carotid artery 15, so that blood flow enters from the inner cavity of the second branch stent 320 into the common carotid artery 15 to ensure smooth blood flow in the left common carotid artery 15. Further, the depth of the recessed area 101 is greater than or equal to 5 mm, and the depth of the recessed area 101 refers to the size of the recessed area 101 in the radial direction of the main stent 100.

Further, please continue to refer to FIG. 2, from the distal end to the proximal end of the main stent 100, the main stent 100 is divided into an anterior segment S 1, a middle segment S2, and a posterior segment S3. Both the first interface and the recessed area 101 are arranged on the middle segment S2. When the stent graft is implanted into the patient's aorta, the anterior segment S1 is placed in the ascending aorta 12, the middle segment S2 is placed in the aortic arch 11, and the posterior segment S3 is placed in the descending aorta 13. The diameter of the posterior segment S3 gradually decreases from the distal end to the proximal end of the main stent 100 to adapt to the change in the diameter of the descending aorta 13. Such arrangement of the anterior arch segment S1 and the posterior arch segment S3 improves the stability for positioning of the stent graft in the aorta. Optionally, the axial length of the anterior segment S1 is 2 cm-10 cm, and the axial length of the posterior segment S3 is 1 cm-20 cm.

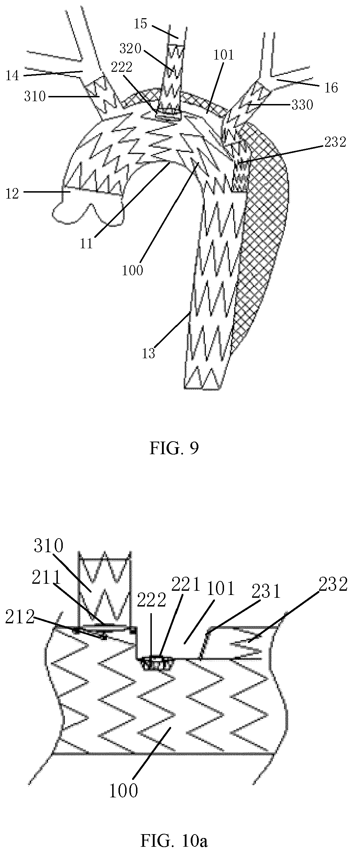

Further, as shown in FIGs 2 and 3, in some embodiments of the present invention, a third interface is further provided on the main stent 100, and the third interface is provided in the recessed area 101 and in communication with the inner cavity of the main stent 100. The first interface, the second interface, and the third interface are sequentially spaced apart along the axial direction of the main stent 100. As shown in FIG. 9, the second interface is used to be detachably connected with a separate second branch stent 320, and the third interface is used to be detachably connected with a separate third branch stent 330.

In some embodiments of the present invention, the first interface may be disposed in an area other than the recessed area 101. When a lesion occurs at the aortic arch 11 and the lesion is located between the innominate artery 14 and the left common carotid artery 15, the first interface is arranged close to the distal end of the main stent 100, so that the first branch stent 310 arranged on the first interface can be implanted into the innominate artery 14, and the recessed area 101 covers the left common carotid artery 15 and the left subclavian artery 16. When the lesion occurs between the left common carotid artery 15 and the left subclavian artery 16, the first interface is arranged close to the proximal end of the main stent 100, so that the first branch stent 310 arranged on the first interface can be implanted into the left subclavian artery 16, and the recessed area 101 covers the innominate artery 14 and the left common carotid artery 15. In some other embodiments of the present invention, the first interface may also be arranged in the recessed area 101, and the stent graft may be applied to the case where the lesion occurs on the lesser curvature side of the aortic arch.

Next, the stent graft provided by the embodiment of the present invention will be described in detail. It should be understood that only one preferred structure of the stent graft is described below, which should not constitute a limitation of the present invention. It should also be understood that in the following description, the case, where the first interface is provided on a side of the main stent except the recessed area 101, and the first branch stent 310 is configured to be implanted into the innominate artery 14, is taken as an example. The skilled person can modify the following description to adapt it to the situation when the first branch stent 310 is implanted in the left subclavian artery 16 or the situation when the first interface is arranged in the recessed area 101.

Please refer to FIGs. 2, 3, 5, 6a and 6b, the first stent body of the main stent 100 includes a first sub-stent body 111 and at least two second sub-stent bodies 112. In some embodiments, the number of the first sub-stent body 111 is one, the at least two second sub-stent bodies 112 are disposed on both sides of the first sub-stent body 111 along an axial direction thereof, and the first sub-stent body 111 and the second sub-stent bodies 112 on both sides enclose the recessed area. In other embodiments, the number of the first sub-stent body 111 is at least two, and the at least two first sub-stent bodies 111 are arranged adjacently and can be connected by a metal rod. The material of the metal rod may be the same material as that of the first sub-stent body 111. The at least two second sub-stent bodies 112 are arranged on both sides of the at least two adjacent first sub-stent bodies 111 along the axial direction thereof. In some embodiments, the radial section of the first sub-stent body 111 may be C-shaped with an opening, and all the openings of the first sub-stent bodies 111 are arranged on the first side. In other embodiments, the cross section of the first sub-stent 111 may be D-shaped, and in this case, the number of the first sub-stent bodies 111 is at least two. The cross section of the second sub-stent body 112 may be circular or elliptical. In this way, when the first membrane 120 covers the outer surfaces of the first sub-stent body 111 and the second sub-stent body 112, the recessed area 101 is formed on the first side of the main stent 100.

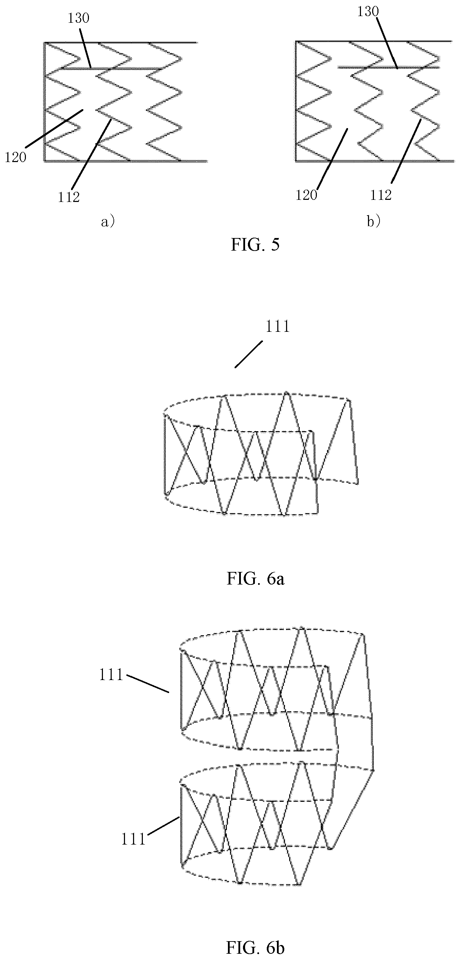

Further, as shown in FIG. 5, each second sub-stent body 112 includes a plurality of V-shaped rods connected end to end in sequence. As shown in FIG. 5a, in the axial direction of the main stent 100, the plurality of V-shaped rods of the same second sub-stent body 112 may have equal lengths. Optionally, as shown in FIG. 5b, in the axial direction of the main stent 100, at least some of the V-shaped rods of the same second sub-stent body 112 may have unequal lengths, and preferably the length of the V-shaped rod gradually increases from the second side to the first side of the main stent 100, so that the rigidity of the second sub-stent body 112 can be increased from the second side to the first side. As a result, the second side is easier to bend and adapt to the lesser curvature side of the aortic arch. In other embodiments, the rigidity of the second sub-stent body 112 may gradually increase from the second side to the first side in other ways. The first sub-stent body 111 can also be arranged in a manner similar to the second sub-stent body 112.

Preferably, the main stent 100 further includes a back support 130 extending along the axial direction of the main stent, and the back support 130 is disposed on the first side of the main stent 100. The main stent 100 may include at least two segments of back support 130, and the two segments of the back support 130 are respectively disposed on both sides of the recessed area 101 along the axial direction thereof to inhibit the shortening of the main stent 100. The back support 130 may be disposed on the second sub-stent body 112, and each back support 130 is connected to at least two of the second sub-stent bodies 112. The back support 130 may also be connected to the first membrane 120.

Please refer to FIGs. 2 and 3, in the axial direction of the main stent 100, the first interface is disposed outside the recessed area 101 and close to the distal end of the recessed area 101. The first interface includes a first window and a first positioning ring 211. The first window is provided on the first membrane 120, the first positioning ring 211 may be formed by bending an elastic metal wire, and the first positioning ring 211 is disposed at the first window and is connected to the first membrane 120. The first positioning ring 211 is used to support the first window so as to open the first window to ensure that blood can flow from the first window into the first branch stent 310, and then into the innominate artery 14.

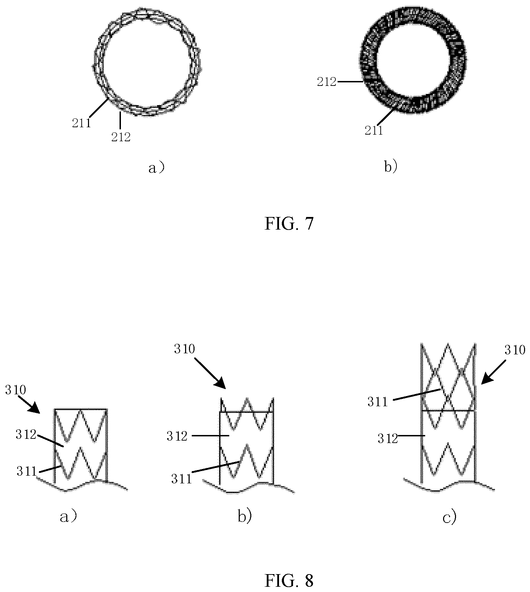

Further, a visualization element (e.g., radiopaque element) 212 is provided on the first interface, so as to determine the position of the first interface during the operation, so that the first branch stent 310 can be accurately implanted into the innominate artery 14. Optionally, the visualization element 212 may be a visualization strip or a visualization wire. In some embodiments, the visualization element 212 is disposed on the first membrane 120 and arranged around the first window. In other embodiments, the visualization element 212 is wound on the first positioning ring 211, as shown in FIG. 7.

Please continue to refer to FIGs. 2 and 3 in combination with FIG. 8, the second stent body of the first branch stent 310 may include a plurality of third sub-stent bodies 311 sequentially arranged along the axial direction thereof. At least one of the third sub-stent bodies 311 located at the proximal end of the first branch stent 310 is covered by the second membrane 312 and inserted into the first window in such a way that it is connected with the first membrane 120 and the first positioning ring 211, so that the axis of the first branch stent 310 is perpendicular to the axis of the main stent 100. In some embodiments, as shown in FIG. 8a, the third sub-stent bodies 311 located at the distal end of the second stent body may be completely covered by the second membrane 312. In some embodiments, at least a part of the third sub-stent body 311 located at the distal end of the second stent body is not covered by the second membrane 312. As shown in FIG. 8b, at least a part of one of the third sub-stent bodies 311 located at the distal end of the second stent body is not covered by the second membrane 312. In some embodiments, as shown in FIG. 8c, at least two of the third sub-stent bodies 311 at the distal end of the second stent body are not covered by the second membrane 312. In this case, the distal end of the second stent body can straddle the bifurcation of the innominate artery 14 to further enhance the anchoring stability of the stent graft without blocking the blood flow at the bifurcation of the innominate artery 14. Optionally, at least a part of the third sub-stent body 311 located at the distal end of the second stent body that is not covered by the second membrane 312 is connected in a non-fixed manner.

When the cross section of the first sub-stent body 111 is C-shaped, the second interface is provided at the opening of the first sub-stent body 111 and includes a second window and a second positioning ring 221. The second window is provided on the first membrane 120, and the second positioning ring 221 is arranged at the second window and connected with the first membrane 120. The second positioning ring 221 is configured to support the second window to open the second window, so that blood flow can smoothly flow out from the second interface. When the second branch stent 320 is inserted into the second interface, and the second branch stent 320 is implanted in the left common carotid artery 15, blood flows into the inner cavity of the second branch stent 320 through the inner cavity of the main stent 100 and the second interface, which ensures a smooth blood flow in the left common carotid artery 15. The second positioning ring 221 can be configured with reference to the first positioning ring 211, which will not be repeated here.

A gap is formed between the second positioning ring 221 and any one of the first sub-stent bodies 111. In this way, since the first membrane 120 is made of an elastic material, the second interface is movable relative to the first sub-stent body 111 so as to adjust the position of the second interface. Specifically, in this embodiment, the minimum distance from any point on the second positioning ring 221 to each of the first sub-stent bodies is greater than or equal to 2 mm.

In an alternative embodiment, when the cross section of the first sub-stent body 111 is D-shaped, and the number of the first sub-stent body 111 is at least two, the second interface is arranged between adjacent two of the first sub-stent bodies 111, and the distance from any point on the second positioning ring 221 to each of the first sub-stent bodies 111 is greater than or equal to 2 mm.

When the second branch stent 320 is provided on the second interface, even if the second interface is not aligned with the left common carotid artery 15, the second branch stent 320 can be implanted into the left common carotid artery 15 by adjusting the position of the second interface in the case that the first branch stent 310 is implanted in the innominate artery 14 because the position of the second interface can be adjusted relative to the first sub-stent body 111. When the third branch stent 330 is further provided on the third interface, the position of the third interface can also be adjusted so that the three branch stents are respectively implanted in the corresponding branch blood vessels, as shown in FIG. 9.

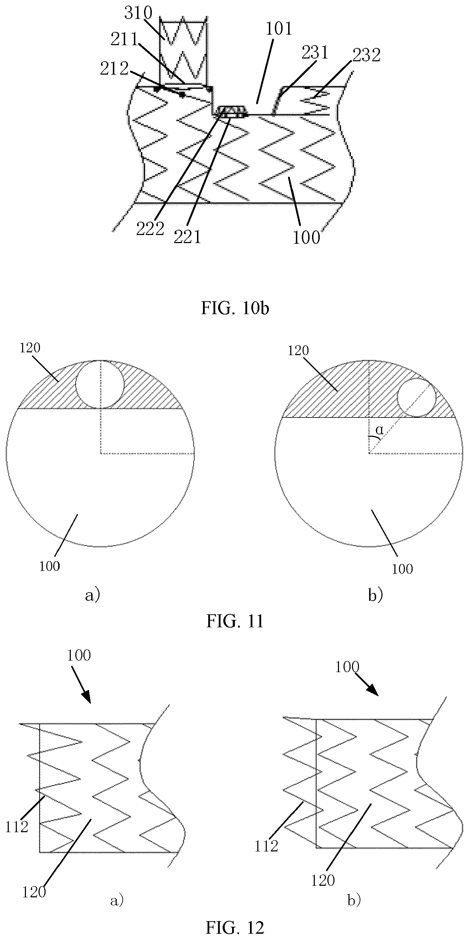

Please refer to FIGs. 10a and 10b in combination with FIG. 9. When the second branch stent 320 is provided on the second interface, the second interface further includes a connector stent 222. The connector stent 222 is arranged at the second window and is connected to both the first membrane 120 and the second positioning ring 221, and the axis of the connector stent 222 is perpendicular to the axis of the main stent 100. The main stent 100 is connected to the second branch stent 320 through the connector stent 222 to increase the connecting area between the second branch stent 320 and the second interface and reduce endoleak.

Optionally, in the axial direction of the connector stent 222, the connector stent 222 has a length of 3 mm-10 mm. As shown in FIG. 10a, in some implementations, one end of the connector stent 222 is disposed at the second window and connected to the first membrane 120 and the second positioning ring 221, and the other end extends into the inner cavity of the main stent 100, so as to serve as a built-in connector stent. In other implementations, one end of the connector stent 222 is disposed at the second window and connected to the first membrane 120 and the second positioning ring 221, and the other end extends to the recessed area 101, so as to serve as a peripheral connector stent.

Please refer to FIG. 9, the third interface includes a third window 231 and an embedded stent 232. The third window 231 is provided on the first membrane 120 of the main stent, and the embedded stent 232 is arranged in the inner cavity of the main stent 100 and extends along the axial direction of the main stent 100. Moreover, one end of the embedded stent 232 is connected with the third window 231, and the other end is in communication with the inner cavity of the main stent 100. The embedded stent 232 includes a distal end and a proximal end. The distal end of the embedded stent 232 is connected to the third window 231, and the inner diameter of the embedded stent 232 gradually decreases from the distal end to the proximal end thereof.

Optionally, in the case that the third branch stent 330 is provided on the third interface, in order to ensure that the third branch stent 330 can be smoothly implanted in the left subclavian artery 16, it shall be ensured, for the stent graft with different sizes, that, after it is implanted in the human aorta, in a cross section of the main stent 100, an angle between the symmetry axis of the first sub-stent body and a line connecting the center of the main stent with the center of the third interface ranges from 0° to 45°, as shown in FIG. 11.

Further, each of the second interface and the third interface are provided with a visualization element, so that the branch stents provided on the second interface and the third interface can be implanted into the corresponding branch arteries. The visualization element provided on the second interface can be arranged with reference to the first interface. The visualization element can be provided on the third window of the third interface and the embedded stent 232 respectively.

In the embodiment of the present invention, the main stent 100, the connector stent 222, and the embedded stent 232 may be separately molded and then connected by stitching or any other suitable ways. In other words, in this embodiment, the main stent 100, the connector stent 222, and the embedded stent 232 may be separated. In other embodiments, the main stent 100, the connector stent 222, and the embedded stent 232 may be integrated. All membranes can be made of bio-non-degradable polymers such as expandable polytetrafluoroethylene (PTFE), polyester (PET), polyester (polyestPE), polyurethane (PU), real silk, etc.

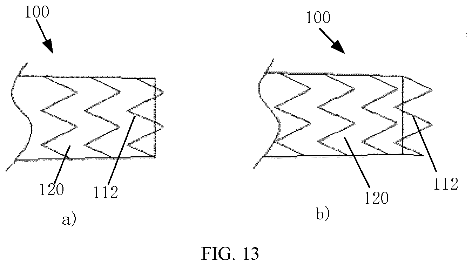

The stent graft provided by the embodiment of the present invention is delivered into the aorta of the patient through a delivery device. Please refer to FIG. 2, the stent graft further includes a restraint wire 400 for restraining the main stent 100 and the branch stents (i.e., the first branch stent 310, the second branch stent 320 and the third branch stent 330). Further, at least a part of the second sub-stent body 112 located at the proximal end or the distal end of the main stent 100 is not covered by the first membrane 120. Specifically, referring to FIG. 5, the apex of the second sub-stent body 112 facing the distal end of the main stent 100 is called the distal apex, and the apex facing the proximal end of the main stent 100 is called the proximal apex. Please refer mainly to FIG. 12, preferably, at least part of the distal apex of a second sub-stent body 112 located at the distal end of the main stent 100 is not covered by the first membrane 120. Similarly, please refer to FIG. 13, at least part of the proximal apex of a second sub-stent body 112 located at the proximal end of the main stent 100 is not covered by the first membrane 120. In this way, the stent graft can be compressed and installed on the delivery device. Optionally, at least a part of the second sub-stent body 112 located at the distal end of the main stent 100 is non-fixedly connected to the first membrane 120, and at least a part of the second sub-stent body located at the distal end of the main stent 100 is non-fixedly connected to the first membrane 120.

Although the present invention is disclosed as above, it is not limited to this. It is apparent that those skilled in the art can make various modifications and variations to the present invention without departing from the spirit and scope thereof. Accordingly, the invention is intended to embrace all such modifications and variations if they fall within the scope of the appended claims and equivalents thereof.

## Claims

1. A stent graft, comprising a main stent, a first interface, a second interface, and a first branch stent; the main stent comprises a first stent body and a first membrane covering the first stent body; the first branch stent comprises a second stent body and a second membrane covering the second stent body;
the first interface is arranged on a side of the main stent and in communication with an inner cavity of the main stent; the first branch stent is arranged on the first interface and extends outward, and an end of the second stent body close to the main stent is at least partially connected to the second membrane in a circumferential direction; a recessed area is formed on a side of the main stent; the second interface is arranged on the recessed area and in communication with the inner cavity of the main stent.

2. The stent graft according to claim 1, wherein the first interface is disposed outside the recessed area.

3. The stent graft according to claim 1, wherein the recessed area has a depth greater than or equal to 5 mm along a radial direction of the main stent.

4. The stent graft according to claim 1, wherein the first interface comprises a first window and a first positioning ring; the first window is provided on the first membrane of the main stent; the first positioning ring is arranged at the first window and configured to support the first window.

5. The stent graft according to any one of claims 1-4, wherein the stent graft further comprises a third interface that is arranged in the recessed area of the main stent and in communication with the inner cavity of the main stent; the first interface, the second interface and the third interface are arranged at intervals along an axial direction of the main stent.

6. The stent graft according to claim 5, wherein the second interface comprises a second window and a second positioning ring; the second window is provided on the first membrane of the main stent; the second positioning ring is arranged at the second window and configured to support the second window.

7. The stent graft according to claim 6, wherein the first stent body comprises one first sub-stent body and at least two second sub-stent bodies; the at least two second sub-stent bodies are arranged on two sides of the first sub-stent body along an axial direction thereof; the first sub-stent body and the second sub-stent bodies on the both sides enclose the recessed area; or
the first stent body includes at least two first sub-stent bodies adjacently arranged and at least two second sub-stent bodies, and the at least two second sub-stent bodies are arranged on two sides of the at least two first sub-stent bodies along an axial direction thereof; the first sub-stent bodies and the second sub-stent bodies on the both sides enclose the recessed area.

8. The stent graft according to claim 7, wherein a gap is formed between the second positioning ring and any one of the first sub-stent bodies; a minimum distance from any point on the second positioning ring to each of the first sub-stent bodies is greater than or equal to 2 mm.

9. The stent graft according to claim 6, wherein the second interface further comprises a connector stent; the connector stent is arranged at the second window and connected with the second positioning ring; an axis of the connector stent is perpendicular to an axis of the main stent.

10. The stent graft according to claim 9, wherein the connector stent has an end connected to the second positioning ring, and a further end extending into the recessed area or the inner cavity of the main stent.

11. The stent graft according to claim 9, wherein the connector stent is extended to a length ranging from 3 mm to 10 mm along an axial direction thereof.

12. The stent graft according to claim 7, wherein the third interface comprises a third window and an embedded stent; the third window is provided on the first membrane of the main stent; the embedded stent is arranged in the inner cavity of the main stent and extends along the axial direction of the main stent; the embedded stent has an end connected to the third window, and a further end connected to the inner cavity of the main stent.

13. The stent graft according to claim 12, wherein the embedded stent comprises a distal end and a proximal end; the distal end of the embedded stent is connected to the third window; an inner diameter of the embedded stent gradually decreases from the distal end to the proximal end thereof.

14. The stent graft according to claim 12, wherein in a cross section of the main stent, an angle between a symmetry axis of the first sub-stent body and a line connecting a center of the main stent with a center of the third interface ranges from 0° to 45°.

15. The stent graft according to claim 7, wherein the main stent has a first side and a second side that are diametrically opposite to each other; the recessed area is provided on the first side;
each of the first sub-stent bodies includes a plurality of V-shaped rods connected end to end in sequence; in the axial direction of the main stent, lengths of the plurality of V-shaped rods of one of the first sub-stent bodies gradually increase from the second side to the first side; and/or
each of the second sub-stent bodies includes a plurality of V-shaped rods connected end to end in sequence; in the axial direction of the main stent, lengths of the plurality of V-shaped rods of one of the second sub-stent bodies gradually increase from the second side to the first side.

16. The stent graft according to claim 7, wherein the main stent has a first side and a second side that are diametrically opposite to each other; the recessed area is provided on the first side; the main stent further comprises a back support extending along the axial direction of the main stent; the back support is arranged on both sides of the recessed area along an axial direction thereof and connected to the second sub-stent body and/or the first membrane.

17. The stent graft according to claim 7, wherein the main stent has a proximal end and a distal end that are opposite to each other; at least a part of the second sub-stent body located at the proximal end and/or the distal end of the main stent is not covered by the first membrane.

18. The stent graft according to claim 7, wherein the main stent has a proximal end and a distal end that are opposite to each other; at least a part of the second sub-stent body located at the proximal end and/or the distal end of the main stent is non-fixedly connected to the first membrane.

19. The stent graft according to claim 5, wherein the stent graft further comprises a second branch stent and/or a third branch stent; the second branch stent is configured to be arranged on the second interface and is detachably connected to the second interface; the third branch stent is configured to be arranged at the third interface and detachably connected to the third interface.

20. The stent graft according to claim 5, wherein the main stent has a proximal end and a distal end that are opposite to each other; the main stent comprises an anterior segment, a middle segment and a posterior segment along a direction from the distal end to the proximal end of the main stent; the first interface and the recessed area are provided on the middle segment, wherein the axial length of the anterior segment has an axial length ranging from 2 cm to 10 cm, and/or the posterior segment has an axial length ranging from 1cm to 20 cm.

21. The stent graft according to claim 20, wherein a diameter of the posterior segment gradually decreases from the distal end to the proximal end of the main stent.

22. The stent graft according to claim 5, wherein a visualization element is provided at each of the first interface, the second interface and the third interface.

23. The stent graft according to claim 1, wherein the second stent body includes a plurality of third sub-stent bodies sequentially arranged along an axial direction of the second stent body;
at least a part of one of the third sub-stent bodies located at a distal end of the second stent body is not covered by the second membrane; or
at least a part of one of the third sub-stent body located at a distal end of the second stent body that is not covered by the second membrane is non-fixedly connected.
